# EUROPEAN PATENT APPLICATION

(11) **EP 4 186 486 A1**
(43) Date of publication of application: **31.05.2023**
(21) Application number: 21210542.3
(22) Date of filing: 25.11.2021
(51) Int. Cl.: A61J 7/00, G16H 20/10, A61J 1/03

(54) **APPARATUS FOR DELIVERING A BLISTER AND SYSTEM COMPRISING THE APPARATUS**

(71) Applicant: Maeusli, Alexandre, 1400 Yverdon-les-Bains (CH)
(72) Inventor: Maeusli, Alexandre, 1400 Yverdon-les-Bains (CH); Hochet, Bertrand, 1073 Savigny (CH); Tharin, Fabrice, 1400 Yverdon-les-Bains (CH)
(74) Representative: Schneiter, Sorin

(57) **Abstract**

The present invention concerns an apparatus for distributing packages comprising medicaments to a patient, wherein said packages are provided as a strip in which said packages are aligned adjacent one another. The apparatus comprises a cutting assembly comprising one or more knives for cutting the strip so as to separate a package from the strip and deliver it to the patient. The cutting device is provided so as to allow safe cutting of the strip and protecting against possible injury due to use of one or more knives in the device. The invention also comprises a system comprising the apparatus and an external platform, which is configured to analyse a photo taken from a package so as to derive the information provided on the package.

## Description

### Technical Field

The present invention generally relates to the delivery of medication to a patient. The invention relates in particular to an apparatus for distributing packages comprising medicaments to a user or patient and to a system comprising said apparatus and an external platform.

### Background Art and Problems Solved by the Invention

The timely accurate administration of medicaments by patients at home has been the subject of significant developments in the last decade. The difficulty of patients that are under a complex treatment regime has been described in large detail, for example in US 2018/0012439. Patients may be under a treatment regime involving from a few to more than 15 different pills or tablets a day, sometimes at different times. Frequently, the elderly patients are concerned, who in addition may suffer from mental conditions, making it even more difficult for the patients to follow the prescribed treatment accurately.

The consequences of patient's failure to adhere to the prescribed treatment regime has also been assessed, and may lead to deterioration of the health of the patient, visits to emergency rooms and preventable hospitalizations. The abuse of prescribed medications that may cause dependencies has also been an issue.

In order to address problems associated with the observation of treatment regimes, pharmacies have packaged drugs in linear rolls or strips of packages, pouches, blisters or sachets, with each sachet containing one or more medicaments to be taken at a particular day or at a particular time of a particular day, wherein the packages are provided in the order corresponding to the administration time, such that the medication to be taken next is contained in the package at the free end of the roll. Furthermore, distributing apparatuses for home use have been provided, which are generally adapted to release the appropriate package at the right date and time. Such apparatuses are described, for example, in US 2020/0323738 A1 and US 2013/0066463 Al, for example.

In particular, different distributing devices have been developed, frequently adapted to a particular type of strips and packages. Indeed, different pharmacies tend to produce their own type of blisters, requiring an apparatus that is adapted to distribute the blisters as produced by that pharmacy. For example, in some cases the blisters in a strip of blisters may be provided with a pre-perforated area, such that an individual blister may be removed simply by tearing the respective blister off, as disclosed in US 2018/0012439 A1. US 2020/0060960 A1, on the other hand, discloses an assembly comprising a rotary knife for separating an individual blister from the strip before delivering the blister on a delivery tray.

It is an objective of the present invention, to provide an apparatus and/or system that is suitable to distribute blisters provided from different manufacturers, preferably even in different countries and using different languages for describing the content of individual blisters. While there are many differences between the labelling of blisters, it is noted that the blister sizes have been harmonized.

One objective of distributing apparatuses for home use is to interact with the patient by producing an alert or reminder once a medication is due for consumption.

Distribution devices as described in the art are of a considerable complexity and hence expensive to manufacture, as they are typically highly automated in order to fulfil their task reliably. It is also noted that different patient populations may require more or less automated devices, depending on their capabilities to handle the distribution apparatus. For example, patients or users that retain most of their physical and mental capacities may require less automation, which would make it possible to offer a less expensive distributer. Indeed, the various patient populations may prefer a system adapted to their respective needs and capacities.

The present invention addresses the problems as set out above. Further objectives and problems addressed by the present invention will become apparent from the description of the aspects and embodiments of the invention herein below.

### Summary of the Invention

Remarkably, present inventors provide a system and/or an apparatus for distributing packages comprising one or more medicament to a user, wherein said a system and/or an apparatus for is capable of reading information contained on the package and to deliver, distribute and/or release a package at an outlet opening of the apparatus to the user at a date and/or time indicated on the package to be administered.

Remarkably, the present inventors further provide a system and/or an apparatus for distributing packages comprising one or more medicament to a user, wherein the apparatus comprises a cutting device for cutting a strip of packages so as to cut said strip of packages and detach a package from the end of said strip.

In first aspect, the invention provides a system for distributing packages comprising one or more medicament to a user, wherein said packages are provided in a strip in which said packages are aligned adjacent one another and/or successively, said strip having a first end and a second end; wherein said system comprises an apparatus comprises a camera, configured to take an image of the package; wherein said system comprises one or more data processing system, configured to determine information contained on said package from said image, said information comprising one or more selected from a date and/or a time of administration indicated on the package.

In an aspect, the invention provides a system for distributing packages comprising one or more medicament to a user, wherein said packages are provided in a strip in which said packages are aligned adjacent one another and/or successively, said strip having a first end and a second end; wherein said system comprises an apparatus comprising: a cavity for storing the strip of packages; a transfer path and an outlet opening, wherein the transfer path is provided for guiding the strip of packages from the cavity towards said outlet opening; a camera, configured to take an image of the package before the package is distributed to the user; wherein said system comprises one or more data processing system, configured to determine information contained on said package from said image, said information comprising one or more selected from a date and/or a time of administration indicated on the package; wherein said system is configured to conduct one or both selected from: (a) producing a communication for informing the user that a package is to be retrieved at the outlet opening of said apparatus at said date and/or time; and (b) automatically transporting said package to and/or out of the outlet opening at said date and/or time; wherein said date and/or time are the date and/or time determined by said one or more data processing systems from the image taken by said camera.

In an aspect, the invention provides a method for operating an apparatus for distributing packages comprising one or more medicament to a user, said method comprising; taking an image of said package; determining information on said package from said image, said information comprising one or more selected from a date and/or a time of administration as indicated on the package, and, delivering, distribute and/or releasing the package at said date and/or time.

In an aspect, the invention provides a method for operating an apparatus for distributing packages comprising one or more medicament to a user, wherein said packages are provided in a strip in which said packages are aligned adjacent one another and/or successively, said strip having a first end and a second end; wherein said apparatus comprises a camera configured to take an image of a package of said strip, said method comprising; taking an image of said package; determining information on said package from said image, said information comprising one or more selected from a date and/or a time of administration as indicated on the package; and, (a) producing a communication for informing the user of a package to be retrieved at the outlet opening of said apparatus at said date and/or time; and, (b) automatically transporting said package to and/or out of the outlet opening (22) at said date and/or time; wherein said date and/or time are the date and/or time determined from the image taken by said camera.

In an aspect, the invention provides an apparatus for distributing packages comprising medicaments to a user, wherein said packages are provided as a strip in which said packages are aligned adjacent one another, said strip having a first end and a second end; wherein said apparatus comprises: an activation organ provided for being used by the user for effectuating the release and/or distribution of one or more packages from said first end of said strip; a storage cavity for storing the strip of packages; a transfer path and an outlet opening, wherein the transfer path provided for guiding the strip of packages from the storage cavity towards said outlet opening; a door and a cutting device comprising one or more knives, wherein said door is provided so as to close said opening and wherein said knives are provided for conducting a piercing and/or cutting movement for cutting said strip of packages and thereby separating one or more packages from said first end of said strip; wherein, said door and said cutting device are provided such that, upon the activation of said activation organ by the user, said door is closed before or simultaneously with said piercing and/or cutting movement.

Further aspects and preferred embodiments of the invention are defined herein below and in the appended claims. Further features and advantages of the invention will become apparent to the skilled person from the description of the preferred embodiments given below.

### Brief Description of the Drawings

**Figure 1** shows a photograph of the front side of an exemplary blister-type package comprising medicaments to be used with the apparatus and system according to embodiments of the invention. Information printed on the front side of the package is visible.
**Figure 2** shows a photograph of an exemplary rolled in strip of blisters with medicaments that can be used with the apparatus and system according to embodiments of the invention.
**Figure 3** is a perspective view of an apparatus according to an embodiment of the invention-
**Figures 4A** **and** **4B** are perspective views of the apparatus shown in Fig. 3, sectioned by a horizontal plane in order to render visible the inside of the apparatus. In Fig. 4B, a strip of blisters is shown in semi-transparent in order to illustrate the position and transport part of the strip in the apparatus.
**Figures 5A and 5B** are front and rear perspective views, respectively, of an assembly comprising a door and cutting assembly of the apparatus shown in Fig. 3.
**Figure 6** is an exploded view of the door and cutting assembly of the device shown in Fig. 3.
**Figures 7A-7E** are perspective views of separate components and sub-assemblies of the door and cutting assembly shown in Fig. 6.
**Figures 8A-8E** are perspective views of different positions of the door and the knives of the door and cutting assembly of the device shown in Fig. 3.
**Figures 9** is a schematic view of the system in accordance with an embodiment of the invention.

Hereinafter, preferred embodiments of the device of the invention are described, in order to illustrate the invention, without any intention to limit the scope of the present invention.

### Detailed Description of the Preferred Embodiments

The present invention concerns an apparatus for distributing individual packages containing medicaments to a user, as well as a system comprising such an apparatus and an external platform that is configured to conduct a variety of functions encompassing data analysis and monitoring adherence to a medication scheme. The user is preferably a patient who requires the medicaments. In some embodiments, the user may be a medically trained assistant, such as a nurse, who uses the apparatus on behalf of a particular patient.

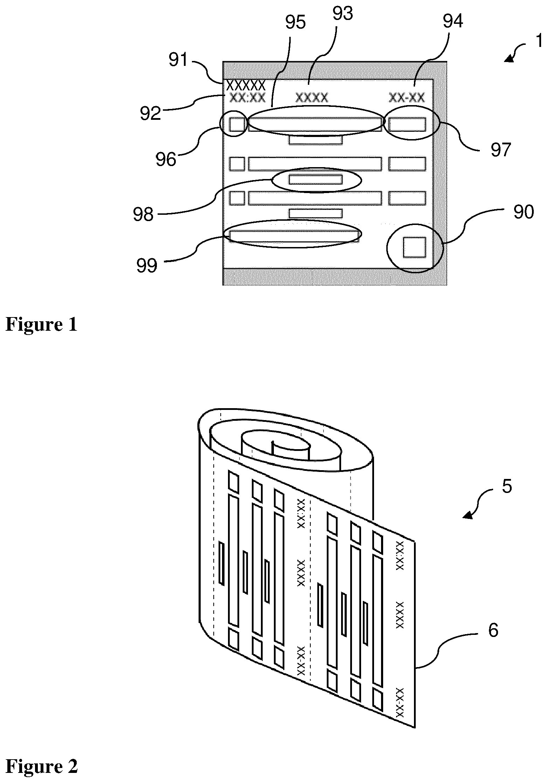

**Figure 1** shows an exemplary package 1 encompassed by the present invention, while **Figure 2** shows a plurality of such packages provided in a continuous strip 5, in which individual packages are aligned adjacent one another.

The individual package 1 may be referred to as a pouch, blister, or small bag, and these meanings are encompassed by the term "package". Each package comprises two polymer sheets one on top of the other, sealed along the two lateral sides of the strip and comprising transvers sealed portions separating the individual packages, such that the sealed portions enclose a space containing one or more medicaments, generally in the form of pills, tablets, capsules and the like. For the purpose of the present specification, the term "item" may be used for referring to a pill, a tablet, a capsule or possibly any other form of administration, preferably solid and preferably for oral administration, of a medicament, as long as the item can be placed in the package without affecting the stability of the medicament and other medicaments potentially present in the package. These medicaments are generally intended for administration by swallowing. As the strip represents a linear chain or string of packages, it comprises a first or free end 6 and a second end, which is not clearly visible in Fig. 2 as the strip 5 is shown rolled up, such that the second end is in the center of the roll and hidden by surrounding packages. The strip 5 is overall flat and has two opposed, first and second sides, defining the two longitudinal sides of the strip, formed by two polymer sheets sealed together or by a folded polymer sheet.

In the strip, the packages are provided in a chronological order, such that the package at the first free end 6 contains the medicaments to be consumed at a given time and date which is closest to the starting date and time of the treatment. In other words, the package at the first end 6 contains the medicaments to be administered next for a given patient, whereas the package at the other end of the strip contains the package with those medicaments to be administered last, considering all medicaments in the strip. The packages are thus preferably chronologically arranged along said strip, wherein the chronological order corresponds to the prescribed administration time and date.

In some embodiments, one package contains the medicaments to be administered at a particular day and/or time, and the subsequent, immediately neighbouring package contains the medicaments to be administered on the next following day or the next following time on the same day, and so forth.

Turning back to Figure 1, each individual package 1 contains information printed on a first or front side of the package. Such information includes the name 91 of the patient to whom the treatment is destined. The information further includes date and time related information, such as the scheduled date 94 and the time 92 of the up-take, as well as the day of the week 93 corresponding to the date.

Each package may comprise one or more items, such as several items of the same medicament or several items encompassing two or more different medicaments. Generally, one or more packages in a strip comprise more than one item. Generally, any one package contains 20 or less items, preferably 15 or less items.

Further, the package contains information related to the medication contained in the very package, such as the active component or medicament name 95, the dosage 97 in one item, and the number of items 96 of the very medicament contained in the package. If there are different medicaments in one package, all of these are mentioned on the package along with information indicated above. In the embodiment shown, each package contains the indication of the color 98 and possibly of the form of the item or items corresponding to a particular medicament.

Preferably, the name 99 of the pharmacy or other provider of the package and/or the strip is also indicated.

In an embodiment, the package may also comprise a QR code 90, which may encode some or all of the precited information, and/or other, additional information.

It is noted that the transversal length of the strip and the lateral dimensions of the packages 1 are generally standardized. However, the way or order the information is printed on the package and the details given are currently not standardized and are generally dependent by the packaging equipment used and the manufacturer producing the strips.

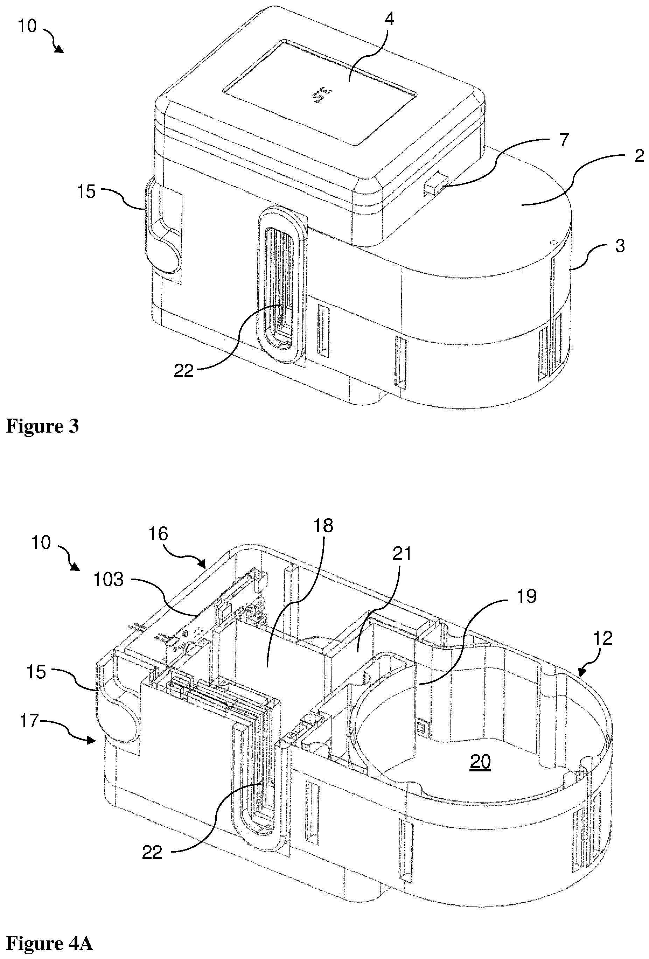

**Figure 3** shows an embodiment of the apparatus 10 for distributing packages or blisters such as the one shown in Fig. 1. As visible in the perspective view, the apparatus comprises a housing 2 defining a reservoir or containment for the strip of packages, an outlet opening 22 where individual packages are delivered to a patient, and an activation organ 15 for being used by the user for effectuating the release and/or distribution of one or more packages. In the embodiment shown, the activation organ is in the form of a hand-actionable lever, which can be pulled down for distributing the package containing the medicaments to be administered. As described further below, the hand-actionable lever 15 is mechanically linked to a cutting device so that the cutting of the strip for the distribution of a package at the free end of the strip is made by exploiting the patient's movement of actuating the lever 15. The invention also encompasses different types of distribution and/or actuation organs, such as a button and in particular a push button, which may be provided to electronically trigger a motor to detach the respective package from the strip. As detailed elsewhere, the apparatus as shown is particularly adapted to be used by patients that are still able to correctly actuate the lever 15 and who are thus to a certain degree capable of living independently and who do not require permanent monitoring by trained health personnel. Such patients preferably still live at their own home.

The apparatus 10 further comprises a door or cover 3, which gives access to the inside of the container and allowing a patient or other user to place a new strip of packages into the apparatus. Instead of a door, a drawing or removable container may be provided to the same end. As visible in Fig. 3, the apparatus preferably comprises a button 7 or possibly a handle for opening the door 3.

Finally, the apparatus of the embodiment shown comprises a display 4 for indicating alerts or other information to the patient.

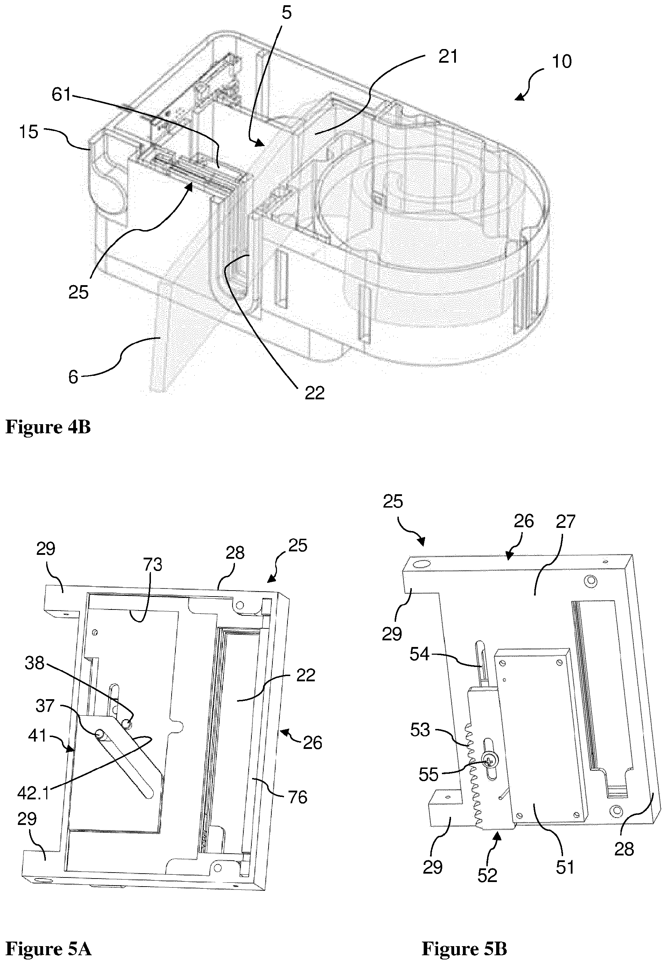

The transversal section through the device shown in **Figures 4A** and **4B** makes it possible to see some of the inner compartments and/or components of the apparatus. Indeed, the apparatus may be conceived as comprising several functional components or units that provide the apparatus as shown. In the embodiment shown, the apparatus is produced by assembling the separately produced components or units. These units encompass the strip-storage and transport unit 12, the electronic control unit 16 and the delivery or distribution unit 17.

The strip storage and transport unit 12 comprises the housing 2 defining the storage cavity 20 in which the strip is stored. In the apparatus of the embodiment shown, the strip is rolled up and stored in the form of a roll (see Figs 2 and 4B) in the cavity 20, which is why the housing 2 defines a hollow cylindrical storage cavity 20 having a substantially circular cross-sectional outline in Fig. 4A.

The invention also encompasses that the strip 5 is folded so as to form a folded stack of packages. In this case, the housing 2 may define a storage space having a substantially rectangular cross-section, for example.

As can be seen in Figs 4A and 4B, the apparatus comprises an inside opening 19 connecting the storage space 20 with a transport path or channel 21, in which the free end 6 of the strip extends towards the outlet opening 22.

The delivery and distribution unit 17 comprises the activation organ 15 and the cutting device for cutting the strip 5 for detaching individual packages 1 at the outlet opening 22. Accordingly, the delivery and distribution unit 17 is associated with the part of the housing of the apparatus that comprises or is at least adjacent to the outlet opening 22.

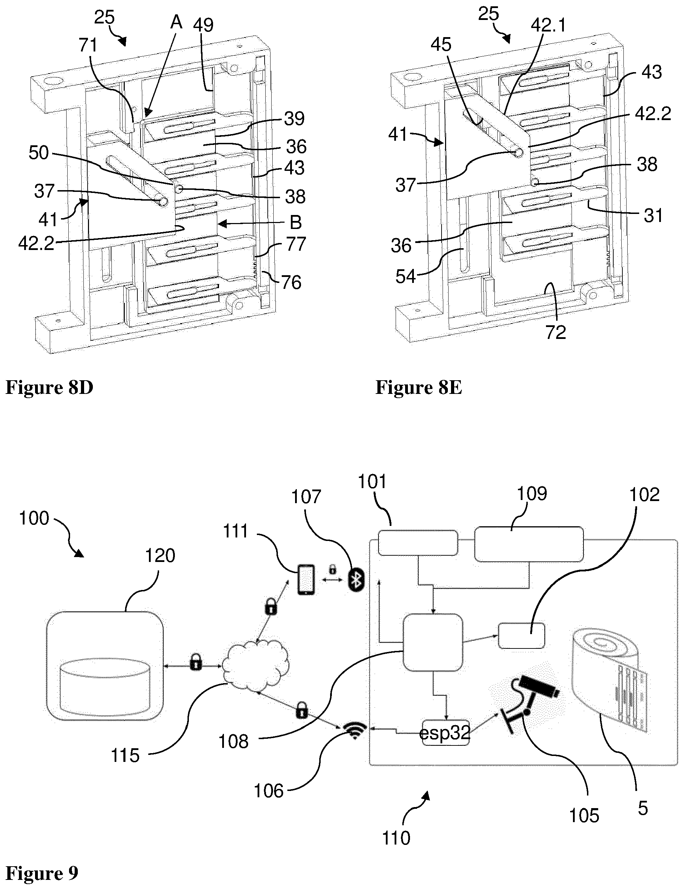

The electronic control unit 16 comprises the electronic components of the device, most of which are provided a printed circuit board (PCB) 103. The electronic components are not visible in detail in Figs 4A and 4B, and will be discussed in more detail with respect to Fig. 9. At this stage it is sufficient to mention that the electronic control unit 16 comprises a data processing entity 108, one or more wireless communication module 106 and a camera 105.

In the apparatus, the camera 105 is positioned so as to be able to take a photo of a package that is to be delivered next, preferably before pushing the package out of the outlet opening 22. The photo is taken as shown in Fig. 1, such that the information printed on the front side of the package is captured on the photo. Accordingly, the apparatus encompasses a space 18 which provides a distance between the camera and the strip 5 in the transport path 21 required to take a satisfactory photo. Furthermore, a light source is preferably provided inside the apparatus, so as to illuminate the strip of packages and in particular the package at the end of the strip to be detached next at the outlet 22.

The apparatus further comprises a motor (not shown) for unreeling the strip of packages and transporting the free end of the strip 5 to the outlet opening 22. Furthermore, the device comprises rollers (not shown) suitable to grip the strip and to draw or push it for unreeling the strip and pushing the free end 6 through the outlet opening when a next package is due for distribution. The motor is preferably under the control of the data processing entity 108 (Fig. 9).

The apparatus of the invention preferably comprises a door and a cutting device, which are provided to safely cut the strip 5 so as to detach the package that is provided at the free end of the apparatus. In particular, the apparatus is configured to open the door and to push the free end 6 out of the door once the medicaments in the package at the free end 6 are due for administration. As discussed further below in more detail, the patient will receive one or more alerts, for example a message or image displayed on the screen 4, an acoustic signal produced by the apparatus and/or an alert via a text message on the patient's mobile device such as a mobile phone. The user can then actuate the activation organ 15 so as to activate the door and cutting assembly, so as to cut through the strip at the very position for detaching the package 1 at the free end 6 of the strip.

The present invention encompasses in particular a door and cutting assembly that makes it possible to provide an apparatus that is safe and that can be used with different types of packages, including packages in strips that do not contain pre-perforated sections for easily detaching individual packages. By providing a door and cutting assembly as described, all types of packages can be detached. Furthermore, the device can be produced at comparatively reduced costs. The door and cutting assembly will be described in the following figures.

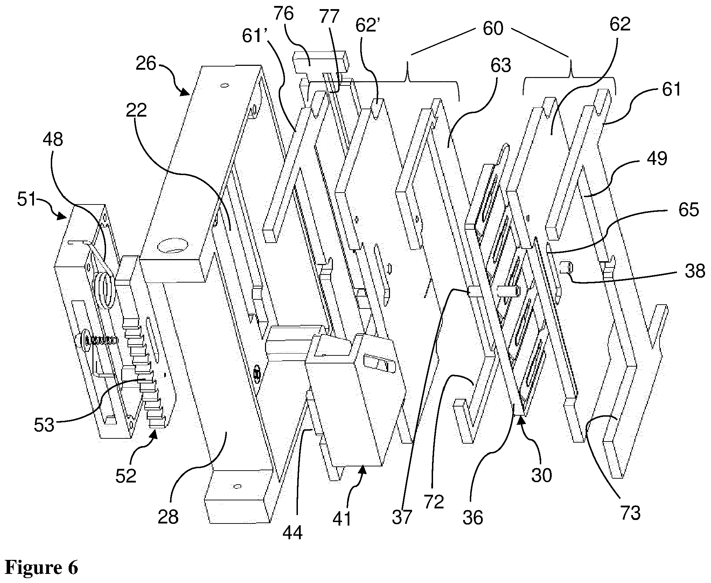

**Figures 5A** and **5B** show perspective views on the two opposed main sides, respectively, of the door and cutting assembly 25. **Figure 6** shows an exploded view of the door and cutting assembly 25, rendering the various components visible.

The assembly 25 comprises a frame element 26, on which the remaining components are attached. The frame 26 is rigidly connected in the housing of the apparatus 10, such that only the mobile parts of the assembly will be able to conduct the movement relative to the housing as required to conduct the cutting of the strip and the closure of the outlet opening 22.

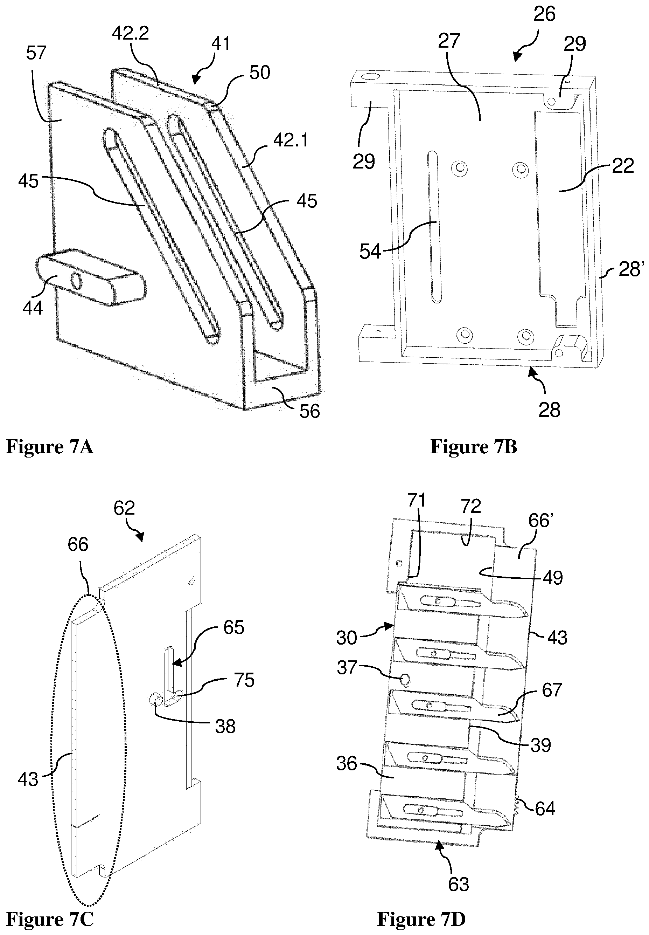

The frame element 26, shown separately in **Figure 7B****,** comprises a flat main plate 27, which comprises a cut-out defining the outlet opening 22, and a rim or frame 28 emerging with a substantially rectangular outline on said main plate, said rim being provided to contain and/or surround the mobile elements, in particular the slider, the door and the cutting device. The frame element 26 further comprises lugs 29 serving as attachment points for mounting the assembly in the apparatus 10, in particular to the housing of the apparatus.

The side of the plate 27 visible in Fig. 5B is arbitrarily referred to as the rear side or second side. When the assembly is mounted, the second side faces towards the inside of the apparatus in the particular embodiment shown (Figs 4A and 4B).

As visible in Fig. 5B, a spring box 51 is attached to the rear side of the main plate. The spring box houses a resilient element 48, preferably a spring (here: a torsion spring, see Fig. 6), provided to act on a transmission element 52 and to urge the same towards a rest position shown in Figs 5A and 5B. The transmission element 52 comprises a coupling structure, which here is provided in the form of a toothed rail 53.

The actuation lever 15 (Figs 4A and 4B) is rigidly connected with a toothed wheel (not shown), geared with the toothed rail 53 shown in Fig. 5B. When a patient pulls down the actuation lever, the toothed wheel rotates and causes the transmission element 52 to conduct a linear motion through the gearing with the toothed rail 53. To this end, the transmission element 52 comprising the toothed rail is slidingly guided by way of a longitudinal slot 54 provided in the main plate 27 of the frame element 26.

A longitudinal connector 44 rigidly connects the transmission element 52 on the second side of plate 27 with a slider 41 provided on the first side of plate 27. The slider 41 and the connector 44 are shown in **Figure 7A****.** Turning back to Figs 5A and 5B, the connector 44 is fixed via fixing screw 55 to the slider 41, such that the connector lies in the slot 54 of the main plate and thus connects mobile parts on both sides of the main plate 27. As can be seen in Fig. 5A, the slider 41 is provided to slide along a straight rim side of the circumferential rim 28 of the frame element 26.

The rim 28 and the main plate 27 form a cavity with an open side for housing the slider 41 and mobile components of the door and cutting assembly 25. As seen in Figs 6 and 7A, the slider 41 comprises a flat base 56 from which two lateral walls 57 emerge. The connector 44 mentioned above is connected rigidly, for example screwed, to one of the two lateral walls. The base and lateral walls provide a frame in which the door and cutting device of the assembly 25 are partially contained. Furthermore, the slider comprises a longitudinal sliding slot 45, provided in the lateral walls 57. Furthermore, the lateral walls form a skewed region 42.1. The slot 45 as well as the skewed region 42.1 serve as abutment regions for driving and/or guiding the movement of the door and cutting device as described elsewhere in more detail.

In Figure 6, the components forming the door 60 and the cutting device 30 of the door and cutting assembly 25 are shown. The door 60 and cutting device 30 are the mobile elements guided by the slider 41 as described below.

The door is formed by a plurality or overall flat frame layers 61-63 and 62', 61', which comprise a closure of the outlet opening 22 but also house the cutting device 30.

From left to right in Fig. 6, the frame layers comprise an outer layer 61', a door plate 62', a frame layer 63, another door plate 62 and another outer layer 61. The cutting device 30 is sandwiched between the two door layers 62, 62', further encased by the frame layer 63. The outer layers 61 and 61' are identical in form and dimensions. The two door layers 62, 62' are mirror symmetrical, as they comprise an L-shaped slot, as visible in **Figure 7C****.** The flat region 66 at one of the sides of the door plate 62 is provided to cover and thus close the outlet opening 22 of the device upon activation of the actuation organ 15.

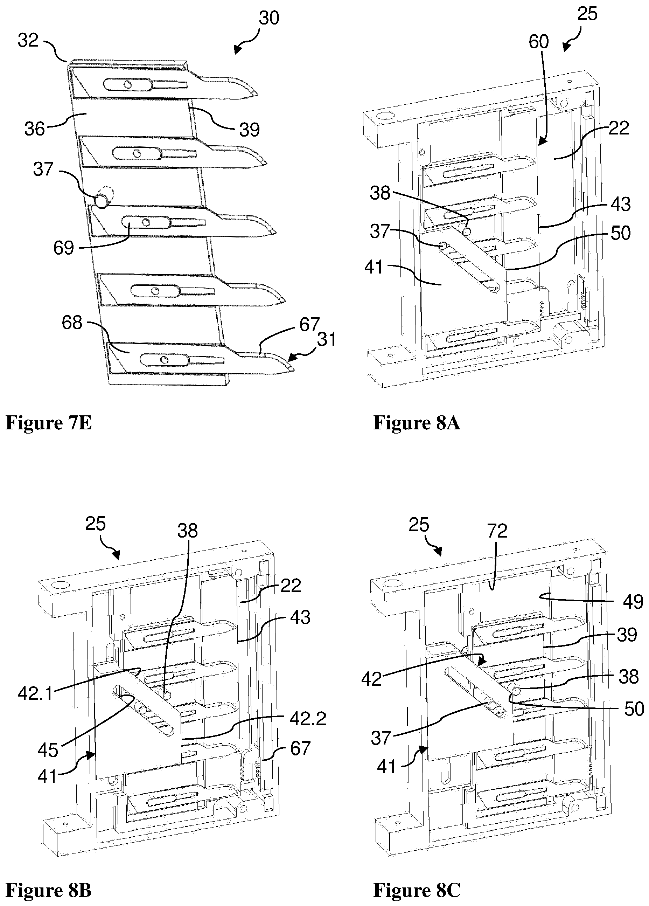

The cutting device 30, shown in **Figure 7E****,** comprises a preferably rectangular attachment plate or knife support 36, on which a plurality of cutting knives 31 are fixed in a detachable manner. Each cutting knife preferably comprises a blade region 67 comprising a cutting edge and a stem 68 for connecting the knife to the attachment plate 36. The stem preferably comprises an opening for attaching the stem on the plate 36, in such a manner that the blade regions emerge free standing from one lateral, preferably straight side 39 of the attachment plate 36.

A pin 37 serving as a pusher is provided on the attachment plate 36. The pin is fixed on the plate 36 so as to extend in a direction that is normal to the plane of the plate 36. Preferably, the knives 31 extend in a direction that is parallel to the plane of the plate 36,

In a preferred embodiment, the knives 31 are scalpel blades, preferably, single-use and/or detachable scalpel blades. Scalpel blades are commercially available, making it possible to produce the cutting device 30 on the basis of readily available materials. The use of scalpel blades makes it also possible to replace the knives conveniently in case the cutting edge blunts.

The knife support 36 preferably comprises connector elements 69, for connecting the surgical blades via the slot in the attachment region 68 of the blade. The connector elements 69, for example in the form of protruding projections, are provided so as to enable the reversible attachment of the knives 31 to the support plate 36 as is known from the attachment of single-use scalpel blades from a scalpel handle.

**Figure 7D** shows the frame layer 63 of the door 60 and the cutting device 30 as disposed when the door 60 is assembled. The frame layer 63 comprises a cut-out 72 of a substantially rectangular outline, in which the knife support plate 36 is placed and housed substantially coplanar with the frame layer 63. The frame layer 63 further defines a closing region or door plate 66', which is aligned with the closing region 66 of the door layers 62, 62' when the layers of the door 60 are assembled. The cut-out 72 of the frame layer 63 further defines a stop or blocking edge 71. A corner 32 of the support plate 36 is shown in abutment against the blocking edge 71, showing that the plate cannot slide within the cut-out 72 in the direction of the blocking edge, but can in principle slide in the perpendicular direction, towards the closing region 66.

At the door region 66', the frame layer 63 further comprises retention teeth or spikes 64, provided to act on the strip 5 and to block it in position during the cutting of the strip, when the door 60 closes. When the door 60 is closed, the teeth 64 squeeze the strip towards the rim part 28' of the frame element 26. As seen in Figs 6, a cutting slot insert or plate 76 is fixed in frame 26, adjacent to the lateral frame rim 28' of the rim 28 (Fig. 7B). The insert 76 comprises a longitudinal slot 77 (Figs 6 and 8D), provided for receiving the tips of the knives 31 during the cutting movement, as will be described below.

When the door 60 is assembled, the various door layers 61-63, 61'and 62' are superimposed and rigidly connected, so as to mechanically behave as a single piece. The cutting device 30 is housed within the door, in particular between the two door layers 62, 62' and within the frame defined by the cut-out 72 of the frame layer 63, such that the knife support 36 is co-planar with the latter. The pins 37 emerging from both sides of the knife support pass through the L-slot 65 in the door layers (Fig. 7C). The door layers also each comprises a pin 38, emerging outwardly.

When assembled as shown in Fig. 5A, the door layers 62, 62' and 63 and the knife assembly are further retained within the two lateral walls 57 of the slider 41. These walls are further substantially co-planar with the two outmost outer layers 61, 61' of the door 30. Indeed, the outer layers 61, 61' also comprise cut-outs 73, sufficiently large to allow and limit the sliding movement of the slider 41.

As also seen in Fig. 5A, when the door and cutting assembly 25 is assembled, the pins 37 of the knife support 36 are guided in the slots 45 of the slider 41. The pin 38 of the door layer is guided on the bearing area 42.1 defined by the skewed ridge of the lateral walls 57 of the slider.

In summary, the pin 38 is rigidly connected with the door 60, whereas the pin 37 is rigidly connected with the cutting device 30.

When a patient pulls down the actuation lever 15, the door 60 closes the outlet opening 22 of the apparatus, the knives pierce the strip in a linear piercing movement in a direction that is substantially normal to the main face of the strip, and then cut the strip in a second linear movement that extends transversally and perpendicularly to the longitudinal extension of the strip. The cutting movements include a first, piercing movement of the knives along the longitudinal extensions of the blades and a second, cutting movement that is substantially perpendicular to the piercing movement. Both movements are conducted in a plane that is parallel or coplanar with the plane defined by the door 60, in particular substantially coplanar with the frame layer 63 of the door.

The operation of the door and cutting assembly 25 and in particular the process of the closure of the outlet 22 and the associated cutting of the strip 5 at the outlet opening is described below with reference to **Figures 8A** to **8E** and Figure 5B. In Figs. 8A-8E, the outer layer 61 and the door layer 62 have been omitted, such that the movement conducted by the cutting device 30 becomes visible. However, the pin 38, rigidly connected to the door layer 62, is shown in Figs 8A-8E, as pin 38 transmits the movement of the slider acting on the door.

As mentioned before, the actuation of lever 15 by a patient results in the linear displacement of the toothed transmission element 52 (Fig. 5B) and the slider 41 that is rigidly connected with the transmission element. During this displacement, the slider 41 moves linearly along slot 54 (Fig. 7B) from the bottom of the frame element 25 as shown in Fig. 8A to the top of the frame element as shown in Fig. 8E. When the patient lets the lever 15 go, the spring 48 drives the slider 41 back to the start or rest position shown in Fig. 8A.

Once the slider 41 moves as shown in Fig. 8B, the border of the slot 45 acts on the pin 37, while the skewed edge 42.1 of the slider acts on pin 38. The door 30 is guided by the frame 28 to move in a direction that is perpendicular to the movement of the slider (from the left to the right in Figs 8A-8E), such that the movement of the slider is transmitted via the pin 38 to the door such that the latter slides in a direction that tends to close the outlet opening 22. As can be seen in Fig. 8B, the closing edge 43 of the door 60 has moved towards the right so as to substantially diminish the opening gap at the outlet opening 22,

During this movement, the cutting device 30 moves along with the door 60, since the pin 37 is guided in parallel in slot 45 of the slider 41. Furthermore, the pin 37 of the cutting device cannot move relative to the door at this stage, as is locked in a recess 75 of the L-slot 65 of the door layer 62 (Fig. 7C). The L-slot is not visible in Figs 8A-8E as the door layer 62 (besides the frame layer 61) has been omitted in these figs. As a further security, a corner 32 of the knife support 36 abuts against the blocking edge 71 provided in the frame layer 63 as shown in Fig. 7D, such that the movement of the entire cutting device 30 is synchronous to the movement of the door 60 in the phase shown in Figs. 8B and 8C.

Fig. 8C shows that the slider 41 has moved further up-wards and the pin 38 is now close to a corner of the ridge 42 defined by the walls 57 of the slider. At this stage, the door has slid so as to nearly completely close the opening 22. It is also noted that the pin 38 of the door has reached the corner 50 of the ridge parts 42.1 and 42.2 and, with the slider moving further towards to the top, the slider will no longer be able to act on the pin 38. On the other hand, the pin 37 of the cutting device has not yet reached the end of guiding slot 45 and will this further be driven by the up-ward movement of the slider 41,

The passage between Fig. 8C to 8Dshows a first part of the relative movement of the cutting device 30 relative to the door assembly 60. As indicated by the arrow A, the corner 32 of the knife support 36 has moved away from the blocking edge 71 provided in the frame layer 63. At this same moment, the pin 37 has left the recess 75 of the L-slot in the door layer 62.

Furthermore, as indicated by arrow B, the edge 39 of the knife support 36 has abutted against abutment rim 49 provided by the door, and in particular by the frame layer 36 of the door.

In this first part of the relative movement of the cutting device relative to the door assembly, the cutting device has moved in the same direction as the door previously, but has moved further than the door, such that the tips of the knives 31 have gone beyond the closing edge 43 of the door 60, as can be seen in Fig. 8D. The tips of the knives now extend into the cutting slot 77 provided in the insert 76 (Fig. 8D). In comparison, in the position illustrated in Figs 7D and 8A-8C, the blades 67 have been remained behind the closing edge 43 and have also remained completely covered between the door layers 62, 62' of the door 60.

The first part of the movement of the cutting device 30 relative to the door assembly 60 represents a piercing movement, in which the knives 61 pierce the strip 5 that is blocked at the outlet opening 22 by the closed door. The piercing movement extends in a direction that is normal to the two main faces of the strip 5.

As described with reference to Fig. 8D and 8E, in a further part of the cutting device 30 relative to the door assembly, the knives will move to cut the strip entirely in a direction that is transverse to the longitudinal strip. In this second part, the knives move along the movement of the slider and no longer perpendicularly to the slider.

As shown in Fig. 8D, the pin 38 of the door has moved past the corner 50 of the slider, such that the slider no longer has any grip on the door other than preventing the door from moving back to the opening position. This is due to the fact that the ridge part 42.2 of the slider 41 extends in parallel to the closing edge 43 of the door and blocks the door from moving. The pin 37 of the cutting device 30 has left the recess 75 of the L-slot 65 and is now free to slide within the longitudinal part of the slot, which extends in parallel to the direction of the slider movement.

As the slider 41 moves from the position in Fig. 8D to the position in Fig. 8E, it acts on pin 37 that is at the end position in slot 45 and thus takes the cutting device 30 along to move in the same direction, which is also predefined by the longitudinal part of the L-slot 65 through which pin 37 extends (Figs 5A and 7C). It is noted that the cut-out 72 in frame layer 63 provides a sufficient free space for allowing the knife support plate to move in that direction. As discussed above, the corner 32 of the knife support 36 is no longer retained by the blocking edge 71 of the frame layer 63. Since the tips of the blades of the knives 31 are past the closing edge 43 of the door, the knives cut the strip that is positioned in the outlet opening 22 and squeezed and blocked by the closing edge of the door 60 and in particular by the retention teeth 64 provided at the closing edge of the door. In the movement of the cutting device from the position in Fig. 8D to the position in 8E, the door 60 does not move, but allows and or guides the cutting movement of the cutting device by way of the L-slot and the shape of the cut-out 72 in which the knife support 36 is retained.

The actuation lever 15 is preferably provided such that once a patient has pulled it down completely, the cutting device 30 has entirely cut the strip 5 and thus released the packaging 1 at the free extremity 6 of the strip. The patient can now release the actuation lever, such that the entire door and knife assembly will move back to the rest position of Fig. 8A under the force of the spring 48 (Fig. 6) acting on the transmission element 52. The movement back to the rest position will be exactly inverse to the closing and cutting movement, in that the knives will first move back to the position where the blades are entirely covered by the door before the door moves so as to open the outlet opening 22.

The apparatus will then remain inactive until being a further time when the medicaments in the next package, now at the free end of the strip 5, are due for administration. At that moment, the apparatus will push the package past the opening 22 and invite the patient to deliver the package by actuating the lever 15 and thereby safely cut the strip as described above.

Although the apparatus of the embodiment shown is provided for manual manipulation of the actuation organ 15, the invention also encompasses that the movement of the door and cutting assembly 25 is driven by a motor. In this case, the actuation may be provided in any form, including a simple button, that is suitable to induce the apparatus electronically to conduct the closing and cutting movement via the electric motor. In this case, a spring for driving the slider back may no longer be required.

It is noted that the apparatus as described herein above is comparatively easy to manufacture while being entirely safe in the operation. This is also due to the fact that the knives used to cut the strip of packages are never exposed to the patient. For example, in case a patient keeps a finger in the opening 22 while actuating lever 15, the door would not be able to close and, consequently, the cutting device would remain blocked relative to the door by structural elements such as the recess 75 in L-slot 65 (Figs. 5A and 7C) and by the blocking edge 71 of the frame layer 63 of the door. Only when the opening 22 is entirely closed by the door 60 can the tips of the knives 31 move past the closing edge 43 of the door.

The apparatus shown in Figs 3 through 8E can be produced at relatively low costs. The apparatus is in particular an apparatus adapted for patients that have still some cognitive capacities so as to be able to manipulate the apparatus and in particular to activate the activation organ 15 and take up the medicaments autonomously. Patients that do not have sufficient autonomy any more may need an apparatus that is easier to manipulate and which will therefore most likely be more expensive to produce.

In some aspects, the present invention also encompasses a system 100 comprising an apparatus 110 for distributing packages containing one or more medicament. The apparatus 110 used in the system may be an apparatus 10 as described herein above, but the system may also be implemented with different apparatuses, as long as they are suitable to deliver individual packages 1 from a strip of packages 5.

**Figure 9** schematically illustrates an embodiment of the system 100. In addition to the apparatus 110, the system 100 preferably further comprises an external platform 120, wherein said apparatus 110 comprises one or more wireless communication module 106, and wherein said apparatus is configured to communicate with said external platform via said wireless communication module 106.

In an embodiment, the apparatus 110 comprises a data processing entity 108 for controlling the operation of the apparatus. The data processing entity 108 preferably comprises one or more selected from a microcontroller, a CPU, suitable ROM and/or RAM memory, a clock and a databus, a source of electrical power or a plug for connection to the power grid, as required for rendering the entity 108 operational. The data processing system 100 and in particular the apparatus 110 comprises a clock and/or firm-or hardware concerning the actual date. The data processing entity 108 may be provided in the form of a printed circuit board (see 103 in Fig. 4A) comprising one or more of the electronic components mentioned above.

In a preferred embodiment, the apparatus comprises a motor provided for acting on said strip 5 so as to transport said strip in a transfer path 21 and to transport the package 1 at the first end 6 of the strip towards said outlet opening 22. The motor is preferably provided for transporting the strip 5 and/or delivering packages at the outlet of the device. Furthermore, the apparatus preferably comprising package distribution detector 101, which produces a signal if a packaging 1 has been delivered at the outlet of the apparatus. In some embodiments, the package distribution detector 101 is a cutting detector, detecting when a patient has actuated the actuation organ 15 so as to cut a package from the strip 5 of packages, for example as described with respect to apparatus 10 shown in Figs 3-4B above.

Preferably, the apparatus comprises a detector or sensor 109 suitable to check if the storage unit 12 (and/or the storage cavity 20) provided for receiving a strip of packages (a "blister roll") has been correctly closed or plugged in. Optionally, this or another detector may also be provided to check if the strip has been correctly inserted in the transport path 21 (Fig. 4A, 4B) so that it can be transported by the aid of the motor and delivered at the outlet opening 22. This sensor is preferably a strip-cavity control detector.

Preferably, one or more selected from the package distribution detector 101, the detector 109 for the correct closure of the storage unit 12, the motor 102, the one or more wireless communication module 106, 107, are in communication with and/or under the control of the data processing entity 108. The entity 108 preferably receives signals from the detectors 101 and 109 and activates the motor when appropriate.

The apparatus 110 preferably comprises one or more camera 105, and one or more wireless communication module 106, 107. The data processing entity 108 is preferably configured to control the camera to take an image of a package before it is distributed to the user and to send said image to the external platform 120 via said wireless communication module 106.

In a preferred embodiment, the apparatus 110 is configured to use said camera (105 to take an image of the package 1 that is at the first end 6 of the strip 5 and which is the package 1 that is next due for delivery and/or administration through the outlet opening 22, or of another package that is a predetermined number of packages away from said package 1 at said first end 6.

The external platform 120 is preferably a server, for example a webserver, and is preferably part of the system 100. The external platform 120 is preferably in communication with, comprises and/or consists of a database, where information is stored. The photo taken by the camera may be sent via the internet or via a mobile network to the external platform 120.

In the embodiment shown in Fig. 9, a separate microprocessor esp32 is used for controlling the camera 105. However, this microprocessor may be considered to be part of the data processing entity 108, as the esp32 microcontroller is also under the control of the data processing entity 108.

The apparatus 110 and/or the system 100 is preferably configured to communicate with a patient via the screen 4 of the apparatus and/or via one selected from a desktop (or personal) computer and a mobile appliance 111, such as a computer tablet or smart phone. For example, the apparatus 110 may communicate via the wireless communication module 107, which here is shown to be a Bluetooth communication module. The invention encompasses wireless communications modules 106, 107, which may independently be selected from Bluetooth, wifi, or LTE (e.g. 4G or 5G), for the communication of the apparatus 110 with the mobile appliance 111 and/or the external platform 120.

The system and/or apparatus may in particular emit communications, such audible signals, alerts and warnings with respect to the administration of medicaments, which may be sent as messages to be displayed on the screen 4 or sent to the mobile appliance 111. Such alerts/messages may indicate, for example, one or more selected from the information, that:
A. a package with one or more medicament is ready for retrieval and administration at the outlet opening and/or that the actuation organ 15 is ready for being activated;
B. a package with one or more medicament is due for administration and retrieval at the outlet within a predefined period of time and/or at a predetermined time in the future; and,
C. a package with one or more medicament has not been retrieved at the outlet opening at the prescribed time.

The system 100 of the invention is preferably configured to take an image of the strip 5 by the camera, to determine information contained on, for example printed or otherwise written or encoded on the package from the image, and to emit a communication for informing the user as mentioned above. At the best knowledge of the inventors, a system, which automatically informs the user on the basis of information printed on the package containing the medicaments is unique and has not yet been disclosed or suggested in the prior art.

In an embodiment, the system 100 is configured to automatically transport a package 1 and/or the first end 6 of said strip 5 through the outlet opening 22 at the date and/or time indicated on the package 1. This position is illustrated in Fig. 4B. The user seeing the package at the outlet 22 is thus aware that the medicaments contained in the package are due for administration. In particular, the communications received by the user from the system and the availability of the package at the outlet 22 substantially coincide and/or occur in a timely and/or organisationally predetermined flow. With the packaging being available, the user may activate the activation organ 15 so as to release the package at the end 6 from the strip. With the apparatus of the embodiment shown in Figs 3-8E, the release is conducted by cutting the strip, but the invention encompasses also that the strip is automatically cut by the apparatus.

In a preferred embodiment, the system 100 is configured to store the effective retrieval date and/or time of packages to users in a database, such as a database contained in the external platform 120. The system 100 is preferably configured to store predetermined extracted information all extracted information in the database.

For the purpose of the present specification, the terms "data extraction" or "extraction of information" are also used to refer to the determination of information contained on the package in accordance with the present invention.

In a preferred embodiment, the system 100 is configured to extract and/or determine at least the date (see numeral 94 in Fig. 1) and/or time (92 in Fig. 1) of intended or scheduled administration as indicated on the package and to inform the user accordingly.

In another embodiment, the system 100 is configured to extract and/or determine one or more additional information contained on said package 1 from said image, said one or more additional information being selected from:
- the day of the week 93 (see Fig. 1) corresponding to the date of administration indicated on the package;
- the name 91 of the patient to whom the treatment contained in the package is destined;
- the active component and/or medicament name 95 of the one or more medicament in the package;
- the dosage 97 of the active component contained in one item of a medicament in the package;
- the number of items 96 of a medicament comprising a given active component;
- the color 98 of the item of a medicament contained in the package;
- the name 99 of the provider or producer of the package and/or the strip;
- a graphical code 90 comprised on the package.

The external platform 120 is preferably configured to conduct automated data extraction so as to determine information contained on a photo taken of the front side of a package 1.

The system 100 comprises computer codes and/or algorithms (hereinafter: software) for analysing the image taken by the camera and for extracting one, several or all of the information mentioned above. It is noted that the information extraction software may in principle be provided in the apparatus 110. In this case, the external platform 120 is no longer necessary or may only function as external database, and the functions of data extraction of the system 100 are accomplished by the apparatus alone. In another embodiment, the overall process of data extraction is partly conducted by the software in the apparatus and partly by the external platform. In yet another embodiment, all data extraction is conducted by the external platform 120, and the apparatus just sends the image as taken to the platform. Preferably, the platform 120 is configured to send part or all information determined by the external platform to the apparatus.

The data extraction preferably comprises several steps or levels, and/or is accomplished by different software or software layers.

As an initial step, the image taken by the camera may be treated so as to improve or enhance subsequent optical character recognition (OCR). Such treatments may include photo enhancement, in particular contrast enhancement and/or image formatting. Such image treatments steps, may, but need not, be conducted by the data processing system 108 contained in the apparatus 110.

In an embodiment, the system of the invention is configured to determine the language of the information 91-99 printed on the package 1. This may be done as part of the OCR software, for example.

In an embodiment, the said one or more data processing system, for determining said information, is configured to conducting optical character recognition (OCR) of characters on said image. From the information obtained by OCR, the system may then determine the information, such as the date and/or time of scheduled administration. In some embodiments, the system is configured to use the information obtained by OCR and to obtain the administration date and/time and to use this information for informing the user as mentioned.

As one may be understood, the timely correct administration of medicaments is preferably warranted by the system of the invention. For this reason, the system of the invention preferably comprises software in addition to information retrieval by OCR. Preferably, the system 100 comprises additional software for the purpose of verifying the extracted information and making sure that the extracted information is correct. Verification preferably involves the assessment of a reliability of the extracted data.

In a preferred embodiment, the said one or more data processing system, for determining said information, is configured to: determining a first or putative information on the basis of said OCR and determining a reliability of the first or putative information. Preferably, the first or putative information is used as the information to be communicated to the user only if said reliability corresponds to or exceeds a minimum required reliability.

Preferably, the system 100 uses the first or putative information and compares it with information that is available to the system. Said data available to the system may include one or more selected from: information related to a particular user as previously determined by the system from images taken by the camera; information that is not related to the user and which is available otherwise to the system; and, information related to a particular user entered independently from the information determined by the system from images taken by the camera.

Preferably, the system 100 is configured to store information associated with a particular apparatus 110, and thus associated with a particular user, in a database (e.g. in platform 120), which database is also part of the system. Such information includes data related to information extracted from previously delivered packages and data concerning the activation of the activation organ 15 by the user (via the cutting detector 101), for example.

Accordingly, once new putative information is extracted by the system from an image of an actual package (or the recent image of a package), the system may compare the congruence between the putative information extracted from the actual package with the information in the database, which information has been extracted from one or more previously distributed package. If there is high congruence, the probability or reliability of the information extracted from the image of the actual package is high or comparatively higher.

For example, if an extracted putative date of administration corresponds to a date that is earlier than a date of administration determined for a previously distributed package, then the reliability can be considered to be low and the putative information is likely wrong. Inversely, in an example where an apparatus distributes one package every day and the putatively determined date of administration is the day following the previously distributed package, then the reliability can be considered to be low. Accordingly, stored information concerning the dates and/or time of packages distributed previously are a preferred source of information for determining the reliability of a putative or first information, for example obtained after OCR.

When assessing the reliability, the system preferably also uses information that is not related to the particular user, but which is generally available to the system, or which can be retrieved via the internet, for example.

For example, when a sequence of letters and in particular a word has been extracted via OCR, the platform preferably comprises algorithms that assesses the extracted word and provides a likely interpretation. For example, week-days may be interpreted so as to relate to the day of a week when administration is scheduled. This information can be verified in an automated manner by comparing with the seven known week-days, the actual day of the week, for example, or by comparing the extracted day of the week with the day of the week corresponding to the extracted date of administration indicated on the package.

As another example, the system 100 may be provided access to a database listing known names of medicaments for determining the names of the medicaments 95 (Fig. 1) as indicated on the package, and/or for comparing the putative name and/or dosage of a medicament as obtained via OCR with existing names of medicaments and existing dosages of these medicaments.

Words may in particular be analysed in view of known names of medicaments, in particular in the language as determined by the system.

In order to account for errors in the OCR algorithm, a similarity analysis may be conducted, where the extracted word is compared with names of medicaments that only differ with respect to a limited number of letters, for example.

In another embodiment, the system 100 and in particular the platform 120 may also make use of information related to a particular user entered and/or available to the system separately and/or independently from the information determined by the system from images taken by the camera. Such data may include information related to the prescribed treatment of a particular patient. Such information may be present in the database, for example entered by a medical doctor treating the patient. Preferably, however, the system of the invention does not need and/or is not given access to information associated with the medical file and/or medical history of individual patient, such that the system is preferably functional without having access to confidential information.

The system 100 preferably runs software for determining the reliability based on information as described. If the said first or putative information corresponds to or exceeds a minimum required reliability, then first or putative information is used as the information communicated to the user.

If the first or putative information has a reliability does not correspond to and/or falls short of said minimum required reliability, then the system is preferably configured to correct the information and/or to enhance the reliability using the information available to the system. Such information available to the system has been exemplified above, in the context of determining the reliability of the information.

The automated adaptation of the putative information in order to improve the reliability preferably involves machine learning algorithms.

In the event that the system 100 is not capable of generating information that has the required minimum reliability level, the system is preferably configured to request external input. In particular, the system is preferably configured not to operate the apparatus 110 and/or to stop and/or block the apparatus, such that no packages are distributed as long as a threshold reliability of the extracted information is reached.

The external input is typically input from a human reviewer, such as a specialist, pharmacist, medical doctor and/or other authorized person. The human reviewer may typically review the image taken and compare the automatically extracted information with the information recognizable to the human from the image. The human reviewer may also request access to the patient file of the particular user. The human reviewer may also induce the system to take a new image of the same package.

Accordingly, the system 100 preferably comprises an interface (not shown), allowing an external reviewer to interact with the system 100 and in particular review the information extracted by the system 100. The human reviewer preferably corrects the putative information having low reliability or confirms the information to be correct if this is the case. The system 100 then preferably considers the reviewed information to be the information that is to be communicated to the user in accordance with the invention. The system preferably makes use of the reviewed information as part of the machine learning algorithms, so as to conduct automated verification and improvement of reliability on the bases of information that has been reviewed by a human specialist.

As mentioned, the system 100 preferably stores extracted information and/or distribution information obtained from the apparatus in a database. The database is preferably external to the apparatus 110, and is preferably on the external platform 120. Preferably, data related to the name of a user and/or patient is stored on a specific, preferably secured module in the database, such that the real name of the patient to which the information pertains is not freely available and separate from other information. For example, the data is stored on a GDPR compliant and certified module of the platform. Information related to the name of a patient can preferably only be accessed if the patient gives the access rights. A restricted circle of persons, such as the patient's doctor, will be able to access the patient's name and the information associated with the patient in the platform 120.

## Claims

1. A system (100) for distributing packages (1) comprising one or more medicament to a user, wherein said packages are provided in a strip (5) in which said packages are aligned adjacent one another and/or successively, said strip having a first end (6) and a second end; wherein said system comprises an apparatus (10, 110) comprising:
- a cavity (20) for storing the strip of packages;
- a transfer path (21) and an outlet opening (22), wherein the transfer path is provided for guiding the strip of packages from the cavity towards said outlet opening;
- a camera (105), configured to take an image of the package (1) before the package (1) is distributed to the user;
wherein said system (100) comprises one or more data processing system (103, 108, 120), configured to determine information contained on said package (1) from said image, said information comprising one or more selected from a date (94) and/or a time (92) of administration indicated on the package (1);
wherein said system (110) is configured to conduct one or both selected from:
(a) producing a communication for informing the user that a package (1) is to be retrieved at the outlet opening (22) of said apparatus at said date and/or time; and,
(b) automatically transporting said package (1) to and/or out of the outlet opening (22) at said date and/or time;
wherein said date and/or time are the date and/or time determined by said one or more data processing systems (103, 120) from the image taken by said camera (105).

2. The system (100) of claim 1, wherein said communication is selected from and alert, an audible signal, a message displayed on a screen (4) of said apparatus, and a message sent to a mobile appliance (111), such as a mobile phone, of said user, and from two or more of the aforementioned.

3. The system (100) of any one of the preceding claims, which is configured to automatically transport a package (1) and/or the first end (6) of said strip (5) through the outlet opening (22) at the date and/or time indicated on the package (1).

4. The system (100) of any one of the preceding claims, wherein said apparatus (10, 110) is configured to use said camera (105) to take an image of the package (1) that is at the first end (6) of the strip (5) and which is the package (1) that is next due for delivery and/or administration through the outlet opening (22), or of another package that is a predetermined number of packages away from said package (1) at said first end (6).

5. The system (100) of any one of the preceding claims, wherein said one or more data processing system (103, 108, 120), for determining said information, is configured to:
- conducting optical character recognition (OCR) of characters on said image;
- determining a first or putative information on the basis of said OCR;
- determining a reliability of the first or putative information;
- using said first or putative information as said information if said reliability corresponds to or exceeds a minimum required reliability.

6. The system (100) of claim 5, which is further configured to request an external input if said reliability does not correspond to and/or falls short of said minimum required reliability, wherein said external input preferably comprises verification of said first or putative information by a human.

7. The system (100) of any one of claim 5 or 6, wherein determining a reliability of the first or putative information comprises comparing said first or putative information with data available to the system.

8. The system (100) of claim 7, wherein said data available to the system includes one or more selected from:
- information related to a particular user as previously determined by the system from images taken by the camera;
- information, such as the actual date and time, that is available to the system but which is not related to a particular user;
- information related to a particular user entered and/or available to the system separately and/or independently from the information determined by the system from images taken by the camera.

9. The system of any one of the preceding claims, wherein said one or more data processing system (103, 108, 120) is configured to determine one or more additional information contained on said package (1) from said image, said one or more additional information being selected from:
- the day of the week (93) corresponding to the date of administration indicated on the package;
- the name (91) of the patient to whom the treatment contained in the package is destined;
- the active component and/or medicament name (95) of the one or more medicament in the package;
- the dosage (97) of the active component contained in one item of a medicament in the package;
- the number of items (96) of a medicament comprising a given active component;
- the color (98) of the item of a medicament contained in the package;
- the name (99) of the provider or producer of the package and/or the strip;
- a graphical code (90) comprised on the package.

10. The system of any one of the preceding claims, which is configured to store the effective retrieval date and/or time of packages to users in a database.

11. The system (100) of any one of the preceding claims, which comprises an external platform (120), wherein said apparatus (10, 110) comprises one or more wireless communication module (106), and wherein said apparatus is configured to communicate with said external platform via said wireless communication module (106).

12. The system of claim 11, wherein said external platform comprises codes and/or algorithms for determining said information from said image, and wherein said platform is configured to send said information to said apparatus.

13. A method for operating an apparatus for distributing packages (1) comprising one or more medicament to a user, wherein said packages are provided in a strip (5) in which said packages are aligned adjacent one another and/or successively, said strip having a first end (6) and a second end; wherein said apparatus comprises a camera (105) configured to take an image of a package of said strip (5), said method comprising;
- taking an image of said package (1);
- determining information on said package (1) from said image, said information comprising one or more selected from a date (94) and/or a time (92) of administration as indicated on the package (1); and,
(a) producing a communication for informing the user of a package (1) to be retrieved at the outlet opening (22) of said apparatus at said date and/or time; and,
(b) automatically transporting said package (1) to and/or out of the outlet opening (22) at said date and/or time;
wherein said date and/or time are the date and/or time determined from the image taken by said camera (105).

14. The method of claim 13, wherein determining information on said package (1) from said image comprises:
- conducting optical character recognition (OCR) of characters on said image;
- determination of a first or putative information on the basis of said OCR;
- determining a reliability of the first or putative information;
- using said first or putative information as said information if said reliability corresponds to or exceeds a minimum required reliability.

15. The system of any one of claims 1-12 and/or the method of any one of claim 13-14, wherein said apparatus comprises an activation organ (15) provided for being activated by the user for effectuating the release and/or distribution of one or more packages from said first end of said strip (5).

16. The system of any one of claims 1-12 and 15, and/or the method of any one of claims 13-15, wherein optical character recognition (OCR) and/or comprises automatic identification of the language of the text on the packaging.

17. An apparatus (10) for distributing packages (1) comprising medicaments to a user, wherein said packages are provided as a strip (5) in which said packages are aligned adjacent one another, said strip having a first end (6) and a second end; wherein said apparatus comprises:
- an activation organ (15) provided for being used by the user for effectuating the distribution of one or more packages from said first end of said strip (5);
- a storage cavity (20) for storing the strip of packages;
- a transfer path (21) and an outlet opening (22), wherein the transfer path provided for guiding the strip of packages from the storage cavity towards said outlet opening;
- a door (60) and a cutting device (30) comprising one or more knives (31), wherein said door is provided so as to close said opening and wherein said knives are provided for conducting a piercing and/or cutting movement for cutting said strip of packages and thereby separating one or more packages from said first end of said strip;
wherein, said door and said cutting device are provided such that, upon the activation of said activation organ (15) by the user, said door is closed before or simultaneously with said piercing and/or cutting movement.

18. The apparatus of claim 17, wherein said door (60) and said one or more knives (31) are provided so as to move sequentially and/or simultaneously in such a manner that said door shields said knives at said opening such that said knives are at no moment exposed at said opening when said device is at rest or during distribution of packages.

19. The apparatus of claim 17 or 19, further comprising a door and cutting assembly (25) connecting said door (60) and said one or more knives (31) mechanically.

20. The apparatus of claim 19, wherein said door and cutting assembly (25) is provided so as to translate a linear or rotational activation movement that is preferably generated upon the activation of said activation organ (15) into a linear movement of said door resulting in the closure of said outlet opening (22).

21. The apparatus of claim 19 and/or 20, wherein said door and cutting assembly (25) is provided so as to translate a linear or rotational activation movement into one or more linear movements of said one or more knives (31) resulting in the cutting of said strip of packages.

22. The apparatus of any one of claims 17-21, wherein said door and cutting assembly (25) comprises a slider (41) guided in said assembly so as to conduct a linear sliding movement upon the activation of said activation organ (15).

23. The apparatus of claim 22, wherein said slider is provided to act on both said door (60) and said one or more knives (31) such that said linear sliding movement results in linear movements of said door and said one or more knives.

24. The apparatus of any one of claims 17-23, wherein said door and cutting assembly (25) comprises a slider (41) guided so as to conduct a linear sliding movement upon the activation of said activation organ (15), such that said linear sliding movement results in first and second movements of said knives a first movement in at least a first direction resulting in the piercing of said strip of packages by said knives and a second movement in at least a second direction resulting in the cutting of strip of packages.

25. The apparatus of any one of claims 17-24, wherein said door is connected to a pusher and/or pin (38), and wherein said door and cutting assembly (25) and in particular said slider (41) is provided to act on said pusher and/or pin so as to close said outlet opening (22).

26. The apparatus of any one of claims 17-24, wherein said one or more knives are connected to a pusher or pin (37), and wherein door and cutting assembly (25) and in particular said slider (41) is provided to act on said pusher and/or pin so as to cause a movement of said one or more knives to pierce and/or cut said strip (5).

27. The apparatus of any one of claims 22-26, wherein said slider comprises first and second bearing regions (42, 45), the first bearing region (42) being provided to act on said door, preferably on said pusher and/or pin (38), and the second bearing region (45) being provided to act on said one or more knives (31), preferably on a second pusher and/or pin (37) connected to said one or more knives (31).

28. The apparatus of any one of claims 22-27, wherein said slider comprises a bearing region (42), provided to act on said door (60), preferably on said a pusher and/or pin (38) connected to said door, said bearing region (42) comprising first and second parts (42.1, 42.2), wherein said first part (42.1) is provided to translate a sliding movement of said slider to a closing movement of said door, and said second part (42.2) is provided so as to keep said door closed and allow said slider to move further and act on said one or more knives (31), preferably on a second pusher and/or pin (37) connected to said one or more knives (31), so as to conduct said piercing and/or cutting movement.

29. The apparatus of any one of the preceding claims, which comprises a knife support (36), wherein said one or more knives are connected on said knife support, and preferably wherein said pusher (37) is provided on said knife support.

30. The apparatus of claim 13, wherein said door (60) comprises an abutment region (49) provided for limiting a movement of said knife support (36) with respect to said door in such a manner that a cutting blade of said knives can extend a predetermined distance beyond a said door (60), preferably beyond an opening and/or closing edge (43) of said door, wherein said abutment region (49) preferably keeps said door and said one or more knives aligned with respect to said opening and/or closing edge (43) of said door.

31. An apparatus (11) for distributing packages (1) comprising medicaments to a user, wherein said packages are provided as a strip (5) in which said packages are aligned adjacent one another, said apparatus comprising:
- a data processing entity (103, 108);
- a camera (105);
- a wireless communication module (106);
wherein said data processing entity controls said camera and is configured to take an image of a package before it is distributed to the user and to send said image to an external platform (120) via said wireless communication module.

32. The apparatus of claim 31, wherein said wireless communication module (106) is a first wireless communication module, and wherein said apparatus comprises a second wireless communication module (107), configured to communicate with a mobile device (91) of the user, and to trigger the generation of an alert and/or reminder in said mobile device.

33. The apparatus of claim 31 or 32, further comprising a package distribution detector (101), capable of detecting when a package has been distributed and/or removed by the apparatus by a user, and to send a signal to said data processing entity (103, 108) when said package has been removed.

34. The apparatus of any one of claims 21-33, wherein said apparatus comprises a cutting device (30) and an activation organ (15), the activation organ being provided for being used by the user for effectuating a cutting operation in which said strip is cut so as to separate one or more packages (1) from said strip and to distribute said one or more packages to the user, wherein said package distribution detector (101) is preferably configured to detect the activation of said activation organ (15) and/or the occurrence of said cutting operation conducted by said user.

35. The apparatus of any one of the preceding claims, further comprising a motor (102), wherein said data processing entity (103, 108) is configured to activate said motor for acting on said strip (5) so as to move said packages from a cavity (20) to an outlet opening (22) of the apparatus, and preferably in the inverse direction.
